# DEMANDE DE BREVET EUROPEEN

(11) **EP 1 980 222 A1**
(43) Date de publication de la demande: **15.10.2008**
(21) Numéro de dépôt: 08290338.6
(22) Date de dépôt: 08.04.2008
(51) Int. Cl.: A61F 2/44, A61F 2/46

(54) **Système pour remplacer un disque entre deux vertèbres**

(30) Priorité: 10.04.2007 FR 0702580
(71) Demandeur: Razian, Hassan, 94240 l'Hay les Roses (FR)
(72) Inventeur: Cavagna, Rémi-Jacques, 56270 Ploemeur (FR); Commarmond, Jacques, 36000 Chateauroux (FR); Robine, Dominique, 75008 Paris (FR); Cortes Moreno, Antonio, 41002 Seville (ES); Hart, Pierre, 93100 Montreuil (FR); Razian, Hassan, 94240 l'Hay-les-Roses (FR); Razian, Salar, 94240 l'Hay-les-Roses (FR)
(74) Mandataire: Flavenot, Bernard

(57) **Abrégé**

La présente invention concerne les systèmes pour remplacer un disque entre deux vertèbres.

Le système selon l'invention comporte une cage 10 constituée d'un corps 11 délimité par deux faces opposées et par une paroi latérale, un guide d'introduction 30 constitué d'un tube 31 dont la section est au plus égale à la section hors-tout du corps 11, le tube 31 comportant une partie 34-1 rectiligne et une partie 34-2 courbe, la portion de paroi intérieure concave 38 de la partie 34-2 ayant une courbure congruente de celle de la surface latérale convexe 20 du corps 11, la portion de paroi 49 du tube 31 opposée à la portion de paroi 38 ayant une forme apte à coopérer par glissement avec la petite base 17 du corps 11, de moyens d'introduction 21 de la cage 10 réalisés dans la partie 34-1 du tube 31, d'une tige poussoir 43, et de moyens 45 pour commander le déplacement de la tige poussoir 43 dans le tube 31.

## Description

La présente invention concerne les systèmes pour remplacer un disque entre deux vertèbres.

Il est connu que, selon les traumatismes subis par une colonne vertébrale, les praticiens sont amenés à effectuer deux sortes d'intervention chirurgicale.

Quand le traumatisme ne concerne qu'un disque intervertébral et que les deux vertèbres consécutives encadrant ce disque sont en bon état, le praticien peut opter pour le remplacement du disque par une prothèse dont la fonctionnalité est sensiblement équivalente à celle du disque originel, c'est-à-dire laissant aux deux vertèbres la possibilité de se déplacer l'une par rapport à l'autre dans des mouvements de rotation et/ou de translation.

En revanche, quand le traumatisme est plus important, le praticien peut choisir de solidariser les deux vertèbres consécutives par ostéosynthèse. Pour ce faire, le disque intervertébral est partiellement ou en totalité détruit et remplacé par une cage intersomatique associée généralement à un élément favorisant l'ostéosynthèse, par exemple un greffon osseux.

Ces cages intersomatiques sont implantables essentiellement par deux voies, l'une dite "antérieure", l'autre dite "postérieure", chacune de ces voies ayant ses avantages et ses inconvénients.

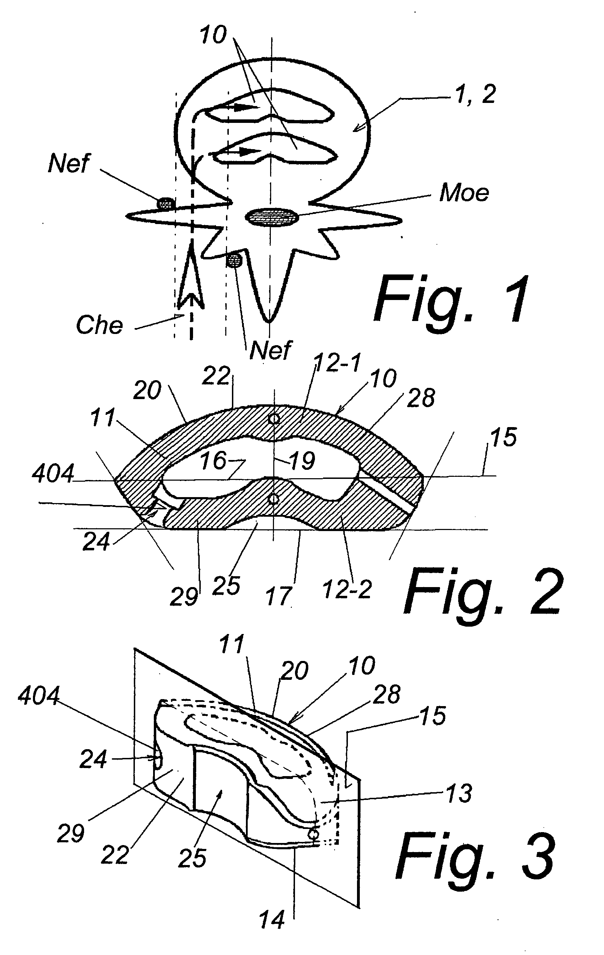

Il semblerait cependant évident que l'implantation par la voie> postérieure soit la plus normale car le chemin Che utilisé par le praticien pour l'intervention chirurgicale est relativement court, comme illustré schématiquement sur la figure 1. Mais elle est très délicate car ce chemin Che passe très près d'éléments essentiels pour la vie du patient, à savoir la moelle épinière Moe et les deux nerfs Nef qui commandent notamment les mouvements du patient.

C'est pour cette raison que certains praticiens optent parfois pour la voie dite "antérieure" qui implique un chemin d'intervention beaucoup plus long, mais en théorie moins dangereux pour le patient. A contrario, le temps d'intervention est plus long que celui par la voie postérieure, ce qui peut présenter un inconvénient pour la santé et la récupération du patient.

Aussi, la présente invention a-t-elle pour but de réaliser un système pour remplacer un disque entre deux vertèbres consécutives, qui permet une implantation par la voie notamment postérieure beaucoup plus facile que celle avec les systèmes similaires de l'art antérieur, et surtout moins dangereuse pour le patient.

Plus précisément, la présente invention a pour objet un système pour remplacer un disque entre deux vertèbres, comprenant au moins une cage intervertébrale et un guide d'introduction de ladite cage pour son introduction entre les deux vertèbres, tel que défini dans la revendication 1 annexée.

D'autres caractéristiques et avantages de l'invention apparaîtront au cours de la description suivante donnée en regard des dessins annexés à titre illustratif mais nullement limitatif, dans lesquels :
La figure 1 représente de façon très schématique l'un des deux processus pour remplacer un disque entre deux vertèbres consécutives et illustre les buts de la présente invention,
Les figures 2 et 3 représentent un mode de réalisation de la cage constituant l'un des deux éléments essentiels du système selon l'invention pour remplacer un disque entre deux vertèbres consécutives, la figure 2 étant une vue en coupe longitudinale de la cage, la figure 3 une vue en perspective cavalière de cette même cage sur laquelle a été matérialisé un plan fictif essentiel pour la définition de la structure de la cage,
Les figures 4A à 4C représentent trois vues en coupe d'un premier mode de réalisation du système pour remplacer un disque entre deux vertèbres selon l'invention, la figure 4A étant une vue en coupe longitudinale, et les figures 4B et 4C étant deux vues en coupe transversale respectivement référencées IVB-IVB et IVC-IVC sur la figure 4A.
Les figures 5, 6 et 7 représentent respectivement trois vues en coupe longitudinale de trois autres modes de réalisation du système selon l'invention pour remplacer un disque entre deux vertèbres, et
Les figures 8 et 9 représentent un perfectionnement aux modes de réalisation du système illustrés sur les figures 4 à 7, la figure 8 étant une vue en coupe longitudinale du système et la figure 9 une vue en perspective d'un détail du mode de réalisation selon la figure 8.

La figure 1 représente de façon très schématique le processus de remplacement d'un disque entre deux vertèbres consécutives 1, 2 par la voie postérieure définie au préambule de la présente description, en passant par le chemin Che à proximité de la moelle épinière Moe et entre les deux nerfs Nef qui commandent notamment les mouvements du patient.

Comme il est dit ci-dessus, la présente invention a pour objet un système pour remplacer un disque entre deux vertèbres consécutives 1, 2, comprenant au moins une cage intervertébrale 10 et un guide d'introduction 30 de la cage pour son introduction entre les deux vertèbres.

Selon une caractéristique de l'invention, par référence aux figures 2 et 3, la cage 10, l'un des deux éléments essentiels du système pour remplacer un disque entre deux vertèbres selon la présente invention, est constituée d'au moins un corps oblong 11 en un matériau biocompatible, par exemple du titane, de l'acier inoxydable, de la fibre de carbone, ou un matériau biodégradable PLLA (Poly L. Lacto Acide) ou un matériau du genre PEEK (Polyéther éther cétone) ou analogue.

Ce corps 11 est défini sensiblement selon un plan longitudinal 15 délimitant fictivement dans ce corps 11 au moins deux première et seconde parties 12-1, 12-2, et délimité par deux faces opposées 13, 14 sensiblement planes et sensiblement parallèles entre elles et par une paroi latérale 22 sensiblement cylindrique réunissant les deux faces planes 13, 14.

La première partie 12-1 du corps 11 forme un secteur sensiblement cylindrique de révolution 28 ayant une surface convexe 20, tandis que la seconde partie 12-2 du corps 11 forme sensiblement un tronc de prisme 29 dont la grande base 16 est située sur le plan longitudinal 15 et est confondue avec la base du secteur cylindrique de révolution 28 et dont la petite base 17 est de forme générale sensiblement plane et sensiblement parallèle au plan longitudinal 15.

Sur la figure 3, qui est une vue en perspective cavalière de la cage 10, le plan longitudinal 15, bien que fictif, a malgré tout été matérialisé pour bien faire ressortir la structure de la cage, et notamment du corps 11, et plus particulièrement les deux parties 12-1, 12-2 de ce corps.

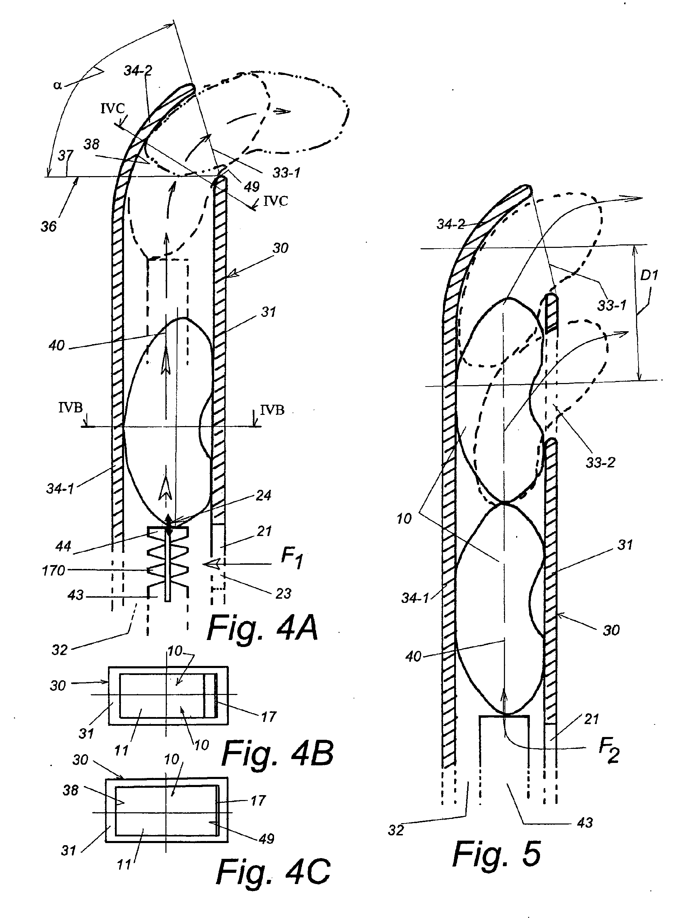

Quant au guide d'introduction 30, figures 4 à 8, l'autre élément essentiel du système selon l'invention, il est constitué par au moins un premier tube 31 ayant une première ouverture proximale 32 et une première ouverture distale 33-1, dont la section intérieure transversale est au plus égale à la plus grande section transversale hors-tout du corps 11 prise dans un plan 19 sensiblement perpendiculaire au plan longitudinal 15 et aux deux faces planes 13, 14 du corps 11, figures 4B et 4C, de préférence sensiblement égale en étant légèrement inférieure.

Le premier tube 31 comporte au moins deux première et seconde parties consécutives 34-1, 34-2. La première partie 34-1 est de forme sensiblement rectiligne et comporte la première ouverture proximale 32 du tube. La seconde partie 34-2 est de forme courbe et comporte la première ouverture distale 33-1. La portion de paroi intérieure concave 38 de la seconde partie 34-2 a une courbure sensiblement congruente de celle de la surface latérale convexe 20 de la première partie 12-1 du corps 11 définie ci-avant, la portion de paroi intérieure 49 de cette seconde partie 34-2 du premier tube opposée à la portion de paroi intérieure concave 38 a une forme apte à coopérer par glissement avec la petite base 17 de la seconde partie 12-2 de ce corps 11.

Le guide 30 d'introduction de la cage 10 comporte en outre au moins des premiers moyens d'introduction 21 de la cage 10 dans la première partie 34-1 du premier tube 31 de façon à amener son plan longitudinal 15 sensiblement confondu avec l'axe 40 de la première partie 34-1 du premier tube 31, une première tige poussoir 43 ou analogue apte à pénétrer dans le premier tube 31 par sa première ouverture proximale 32, et des premiers moyens 45 pour commander le déplacement de la première tige poussoir dans le premier tube 31.

De façon avantageuse, pour faciliter la rotation de la cage 10 dans la seconde partie 34-2 du premier tube 31 comme il sera explicité ci-après, la portion de paroi latérale 22 du corps 11 correspondant à la petite base 17 de la seconde partie 12-2 de ce corps 11 comporte, sensiblement dans sa partie médiane, une gorge 25, figures 2 à 8.

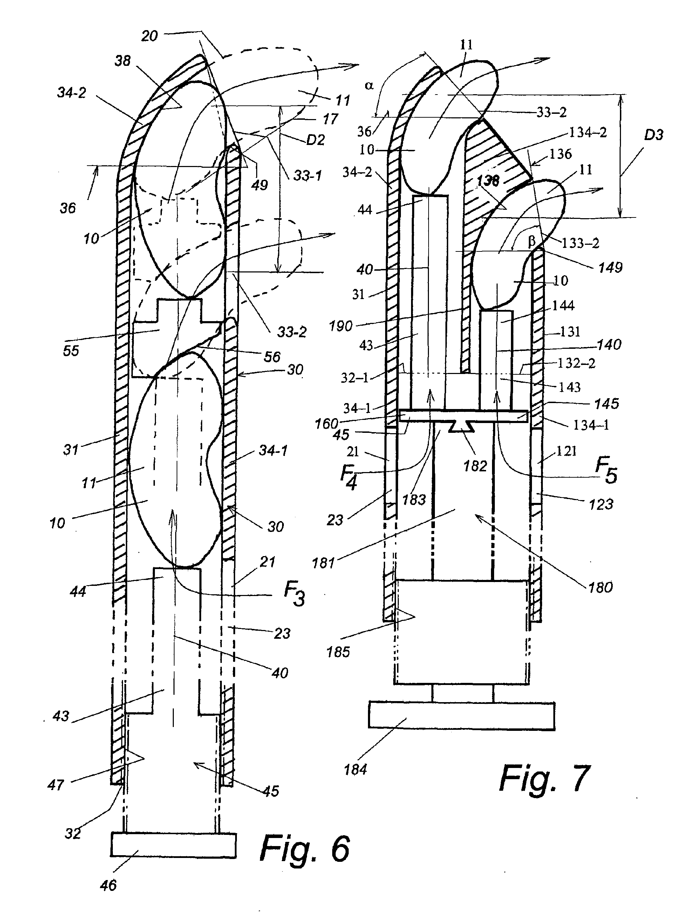

De façon préférentielle, figures 4 à 8, les premiers moyens d'introduction 21 de la cage 10 dans la première partie 34-1 du premier tube 31 comportent une fenêtre 23, 123 réalisée dans la paroi de la première partie 34-1 du premier tube 31, la longueur de cette fenêtre 23, 123 selon l'axe 40 de la première partie du premier tube étant avantageusement au moins égale à la longueur hors-tout maximale du corps 11 et sa largeur étant déterminée pour permettre l'introduction du corps dans le tube 31 comme indiqué par exemple par les flèches F₁ sur la figure 4A ; F₂ sur la figure 5 ; F₃ sur la figure 6 et F₄ et F₅ sur la figure 7.

De façon avantageuse, le système comporte en outre des moyens d'accrochage amovible 24 entre l'extrémité pénétrante 44 de la première tige poussoir 43 et le corps 11, schématiquement illustrés sur la figure 4A.

Dans une réalisation possible, ces moyens d'accrochage amovible 24 entre l'extrémité pénétrante 44 de la première tige poussoir 43 et le corps 11 sont constitués par au moins deux éléments complémentaires détachables, respectivement un élément femelle taraudé et un élément mâle fileté complémentaire apte à être vissé dans l'élément femelle taraudé, l'un de ces éléments étant monté en coopération avec le corps 11 et l'autre avec la première tige poussoir 43.

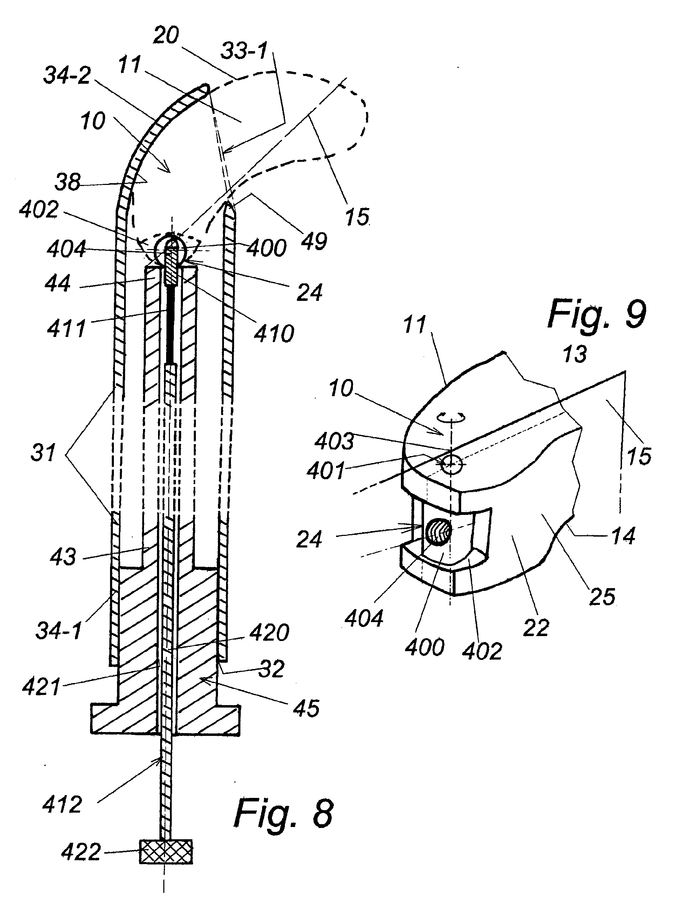

Les figures 8 et 9 représentent un mode de réalisation avantageux de ces deux éléments complémentaires détachables, selon lequel l'élément femelle taraudé est constitué d'un maneton 400 disposé dans une cavité ouverte 402 réalisée dans le corps 11, de moyens 401 pour monter ce maneton en rotation autour d'un axe 403 sensiblement contenu dans le plan longitudinal 15 du corps 11 et agencé avantageusement de façon qu'il soit perpendiculaire à l'axe longitudinal 40 du premier tube 31 quand la cage 10 est dans le premier tube 31, et d'un orifice taraudé 404 réalisé dans ce maneton 400.

Quant à l'élément mâle fileté complémentaire, il est constitué, figure 8, d'un embout fileté rigide 410 complémentaire de l'orifice taraudé 404, pour être apte à y être vissé, d'un lien 411 souple en flexion et rigide en torsion, comme une lame, une tresse métallique ou analogue, dont une extrémité est solidaire de l'embout fileté 410, et de moyens 412 montés en coopération avec l'autre extrémité du lien 411 pour commander le lien en au moins l'un des deux mouvements suivants : rotation, translation.

Selon ce mode de réalisation, ces moyens 412 montés en coopération avec l'autre extrémité du lien 411 pour commander le lien en rotation et/ou translation sont constitués par exemple par une tige de commande 420 montée dans une percée longitudinale 421 réalisée dans la première tige poussoir 43, de façon que cette tige de commande 420 puisse se translater et pivoter librement dans cette percée 421, l'extrémité libre 422 de la tige de commande 420 émergeant de la tige poussoir 43 pour pouvoir être commandée en translation et/ou rotation, par exemple au moyen d'une molette ou analogue ou avec un outil adapté.

Dans une réalisation possible, comme illustré schématiquement sur la figure 6, les premiers moyens 45 pour commander le déplacement de la première tige poussoir 43 dans le premier tube 31 sont constitués par des moyens 46 pour entraîner en rotation la première tige poussoir 43 par rapport au premier tube 31 et des moyens 47 pour transformer le mouvement de rotation de la première tige poussoir 43 en son mouvement de translation.

Ces derniers moyens 47 sont par exemple constitués, comme illustré, d'une partie filetée de la tige poussoir 43, montée vissée dans une partie taraudée de la paroi intérieure du premier tube 31, avantageusement à proximité de son extrémité proximale 32.

De façon préférentielle, figure 5, le premier tube 31 comporte en outre au moins une seconde ouverture distale 33-2 réalisée dans la portion de paroi de sa première partie 34-1 qui est prolongée par la portion de paroi intérieure 49 de sa seconde partie 34-2 apte à coopérer par glissement avec la petite base 17 de la seconde partie 12-2 du corps 11, la distance D1 séparant cette seconde ouverture distale 33-2 et la première ouverture distale 33-1 étant sensiblement égale à la longueur hors-tout maximale du corps 11.

De façon possible, dans le mode de réalisation selon les figures 4A à C, et même éventuellement la figure 5, le système peut comporter en outre (figure 6) un poussoir intermédiaire 55 apte à être introduit entre deux première et seconde cages 10 introduites successivement dans le premier tube 31, la distance D2 entre les première et seconde ouvertures distales 33-1, 33-2 étant alors sensiblement égale à la somme des longueurs hors-tout maximales du corps 11 et du poussoir intermédiaire 55 prise selon l'axe 40 de la première partie 34-1 du premier tube 31, la face 56 du poussoir intermédiaire 55 apte à venir en contact avec la seconde cage 10 présentant une forme courbe sensiblement congruente de la surface latérale convexe 20 du corps 11.

Dans les modes de réalisation illustrés, figures 4 à 8, la seconde partie 34-2 du tube 31 de forme courbe est inscrite dans un dièdre imaginaire 36 d'angle au sommet α (figures 4A et 7), β (figure 7) non nuls, les deux plans du dièdre 36 se situant respectivement sensiblement dans le plan de jonction 37 des deux première et seconde parties 34-1, 34-2 du premier tube 31 et dans le plan de la première ouverture distale 33-1, son arête étant sensiblement confondue avec un bord de la première ouverture distale, la valeur de l'angle au sommet α, β étant comprise entre sensiblement 20 et 90 degrés.

Comme illustré sur la figure 7, le guide d'introduction 30 peut avantageusement comporter au moins un second tube 131 ayant une seconde ouverture proximale 132-2 et une seconde ouverture distale 133-2, dont la section intérieure transversale est sensiblement complémentaire de la plus grande section transversale hors-tout du corps 11 d'une cage 10 identique ou non à celle qui est introduite dans le premier tube 31, prise dans un plan 19 sensiblement perpendiculaire au plan longitudinal 15 et aux deux faces planes 13, 14, ce second tube 131 comportant au moins deux première et seconde parties consécutives 134-1, 134-2, la première partie 134-1 étant de forme sensiblement rectiligne et comportant la seconde ouverture proximale 132-2, la seconde partie 134-2 étant de forme courbe et comportant la seconde ouverture distale 133-2, la portion de paroi intérieure concave 138 de la seconde partie 134-2 du second tube 131 ayant une courbure sensiblement congruente de celle de la surface latérale convexe 20 de la première partie 12-1 du corps 11, la portion de paroi intérieure 149 opposée à la portion de paroi intérieure concave 138 de cette seconde partie 134-2 du second tube ayant une forme apte à coopérer par glissement avec la petite base 17 de la seconde partie 12-2 du corps.

Selon ce dernier mode de réalisation, le système comporte aussi des seconds moyens d'introduction 121 de la cage 10 dans la première partie 134-1 du second tube 131 de façon à amener son plan longitudinal 15 sensiblement confondu avec l'axe 140 de la première partie 134-1 du second tube 131, une seconde tige poussoir 143 apte à pénétrer dans le second tube 131 par son ouverture proximale 132-2, et des seconds moyens 145 pour commander le déplacement de la seconde tige poussoir 143 dans le second tube 131.

Selon ce dernier mode de réalisation, figure 7, les premier et second tubes 31, 131 ont une partie 190 de paroi latérale commune et sont agencés entre eux de façon que la seconde ouverture distale 133-2 du second tube 131 et la première ouverture distale 33-1 du premier tube 31 soient décalées l'une de l'autre suivant une direction parallèle aux axes longitudinaux 40, 140 de la première partie des premier et second tubes, d'une valeur D3 au moins égale à la section hors-tout transversale la plus grande du corps 11 d'une cage 10 prise dans le plan 19 sensiblement perpendiculaire au plan longitudinal 15 défini précédemment en regard des figures 2 et 3.

Selon le mode de réalisation illustré sur la figure 7, qui permet de remplacer un disque intervertébral simultanément par deux cages 10, les premiers moyens 45 pour commander le déplacement de la première tige poussoir 43 dans le premier tube 31 et les seconds moyens 145 pour commander le déplacement de la seconde tige poussoir 143 dans le second tube 131 comportent une embase 160, des moyens pour solidariser cette embase 160 avec les deux extrémités respectivement des deux tiges poussoirs 43, 143 opposées à leurs extrémités pénétrantes 44, 144 et des moyens 180 pour commander la translation de l'embase 160. Des modes de réalisation de ces derniers moyens 180 ne seront pas spécifiquement décrits ici car ils se déduisent sans aucune difficulté des réalisations des moyens 45 décrits précédemment notamment en regard de la figure 6.

Il est malgré tout signalé que ces moyens 180 comportent très schématiquement, figure 7, une came poussoir 181, des moyens 182 pour lier en rotation libre une première extrémité 183 de la came 181 avec l'embase 160, des moyens 184 pour entraîner en rotation la came poussoir 181 par rapport aux deux premier et second tubes 31, 131 et des moyens 185 pour transformer le mouvement de rotation de la came poussoir 181 en son mouvement de translation.

Il est aussi possible que, pour certaines applications, l'extrémité pénétrante 44, 144 d'au moins l'une des première et seconde tiges poussoirs 43, 143 soit avantageusement agencée pour pouvoir subir, sur au moins une longueur déterminée non nulle 170 (figure 4A), une déformation en courbure, par exemple en présentant une structure du type serpent, comme représenté sur cette figure 4A.

Bien entendu, en plus du corps 11, la cage 10 peut comporter d'autres éléments qui sont bien connus dans ce domaine, comme des picots d'accrochage sur au moins l'une des deux faces opposées planes 13, 14, un ou des logements pour des greffons osseux ou analogues, des repères radio-opaques quand le corps est en un matériau radio-transparent comme par exemple en PEEK ou analogue, etc... Tous ces éléments sont bien connus en eux-mêmes et ne seront pas plus amplement décrits ici dans l'unique souci de simplifier la présente description.

Le système selon l'invention s'utilise de la façon suivante, décrite plus particulièrement en faisant référence au mode de réalisation selon les figures 8 et 9. L'utilisation du système selon les modes de réalisation illustrés sur les figures 4 à 7 s'en déduira sans aucune difficulté pour un homme du métier.

Ceci étant précisé, le praticien pratique une incision dans le dos du patient pour créer un chemin d'introduction Che qui passe entre les deux nerfs Nef (figure 1). Il introduit ensuite le guide d'introduction 30 de façon que la seconde partie 34-2 du tube 31 pénètre à l'intérieur du corps du patient jusqu'à ce que l'ouverture distale 33-1 se trouve au niveau de l'espace entre les deux vertèbres dans lequel doit être placée une cage intersomatique 10.

Il peut tout d'abord visser l'embout fileté 410 dans l'orifice taraudé 404 après avoir passé la tige de commande 420 dans la percée 421 réalisée dans la tige poussoir 43. Cet ensemble réalisé, il introduit la cage 10, par exemple par l'extrémité proximale 32 du guide d'introduction 30 et la pousse tout d'abord dans la partie rectiligne 34-1 du tube 31 au moyen de la tige poussoir 43.

On peut supposer que l'extrémité pénétrante 44 de la tige poussoir 43 reste constamment au contact du corps 11 de la cage 10, notamment grâce à une légère traction sur la tige de commande 420 ou grâce aux frictions exercées par le corps 11 sur la paroi intérieure du tube 31.

A partir d'un certain niveau d'introduction de la cage 10 dans le tube 31, la tige poussoir 43 peut être vissée dans l'extrémité proximale du tube, ce qui lui permet de subir une rotation et une translation dans le sens de l'extrémité proximale 32 vers l'extrémité distale 33-1 du tube 31.

Quand la cage 10 arrive au niveau de la seconde partie 34-2 du tube 31, son déplacement subit une déviation due au fait que sa paroi convexe 20 vient glisser sur la paroi intérieure concave 38 de la seconde partie 34-2 du tube 31. En continuant à pousser la cage au moyen de la tige poussoir, le praticien introduit la cage 10 entre les deux vertèbres jusqu'à une position comme celle représentée sur la figure 1, par exemple en partie supérieure.

Le praticien peut parfaire la position de la cage au moyen de la tige de commande 420 qui est, comme mentionné précédemment, liée au corps 11 par les deux éléments d'accrochage mâle-femelle. Si le praticien juge que l'emplacement de la cage n'est pas totalement satisfaisant, il peut ramener la cage dans le tube 31 en exerçant une traction sur la tige de commande 420, puis repositionner la cage de façon plus satisfaisante par le même processus que décrit ci-dessus.

Si le praticien juge que la cage est bien positionnée, il peut, par une rotation de dévissage de la tige de commande 420, désolidariser l'embout 410 du maneton 400. Ce dévissage est rendu possible par le fait que le lien 411 décrit ci-avant présente une souplesse en flexion et une rigidité en torsion.

Lorsqu'une première cage a été positionnée comme décrit ci-dessus, le praticien peut positionner une seconde cage, par exemple en dessous de la première comme illustré sur la figure 1, de la même façon que décrit ci-dessus, mais en ayant retiré le guide d'introduction d'une quantité sensiblement égale à la distance qui doit séparer les deux cages implantées entre les deux vertèbres 1 et 2.

Les modes de réalisation selon les figures 6 et 7 fonctionnent de la même façon que celle décrite ci-dessus pour le mode de réalisation selon la figure 8, mais présentent l'avantage de permettre le remplacement d'un disque intervertébral avec deux cages implantées simultanément. Le fonctionnement de ces deux modes de réalisation ne sera donc pas plus amplement développé ici.

## Revendications

1. Système pour remplacer un disque entre deux vertèbres consécutives (1, 2), comprenant au moins une cage intervertébrale (10) et un guide d'introduction (30) de ladite cage pour son introduction entre les deux vertèbres, **caractérisé par le fait que** :
• ladite cage (10) est constituée d'au moins un corps oblong (11) défini sensiblement selon un plan longitudinal (15) délimitant fictivement dans ledit corps (10) au moins deux première et seconde parties (12-1 ; 12-2), ce corps étant en outre délimité par deux faces opposées (13, 14) sensiblement planes et sensiblement parallèles entre elles et par une paroi latérale (22) sensiblement cylindrique réunissant les deux dites faces planes,
* la première partie (12-1) du corps (11) formant un secteur sensiblement cylindrique de révolution (28) ayant une surface convexe (20),
* la seconde partie (12-2) du corps (11) formant sensiblement un tronc de prisme (29) dont la grande base (16) est située sur ledit plan longitudinal (15) et confondue avec la base du secteur cylindrique de révolution (28) et dont la petite base (17) est de forme générale sensiblement plane sensiblement parallèle au plan longitudinal (15), et que
• ledit guide d'introduction (30) est constitué par au moins :
* un premier tube (31) ayant une première ouverture proximale (32) et une première ouverture distale (33-1), dont la section intérieure transversale est au plus égale à la plus grande section transversale hors-tout du dit corps (11) prise dans un plan (19) sensiblement perpendiculaire au dit plan longitudinal (15) et aux deux faces planes (13, 14), ledit premier tube (31) comportant au moins deux première et seconde parties consécutives (34-1, 34-2), la première partie (34-1) étant de forme sensiblement rectiligne et comportant la première ouverture proximale (32) du dit tube, la seconde partie (34-2) étant de forme courbe et comportant la première ouverture distale (33-1) du dit tube, la portion de paroi intérieure concave (38) de la seconde partie (34-2) du premier tube (31) ayant une courbure sensiblement congruente de celle de la surface latérale convexe (20) de la première partie (12-1) du dit corps (11), la portion de paroi intérieure (49) opposée à la portion de paroi intérieure concave (38) de cette seconde partie (34-2) du premier tube ayant une forme apte à coopérer par glissement avec la petite base (17) de la seconde partie (12-2) du dit corps,
* des premiers moyens d'introduction (21) de ladite cage (10) dans la première partie (34-1) du premier tube (31) de façon à amener son plan longitudinal (15) sensiblement confondu avec l'axe (40) de ladite première partie (34-1) du premier tube (31),
* une première tige poussoir (43) apte à pénétrer dans ledit premier tube (31) par sa première ouverture proximale (32), et
* des premiers moyens (45) pour commander le déplacement de la première tige poussoir dans ledit premier tube (31).

2. Système selon la revendication 1, **caractérisé par le fait que** la portion de paroi latérale (22) du dit corps (11) correspondant à la petite base (17) de sa seconde partie (12-2) comporte, sensiblement dans sa partie médiane, une gorge (25).

3. Système selon l'une des revendications 1 et 2, **caractérisé par le fait que** les premiers moyens d'introduction (21) de ladite cage (10) dans la première partie (34-1) du premier tube (31) comporte une fenêtre (23, 123) réalisée dans la paroi de la première partie (34-1) du premier tube (31), la longueur de ladite fenêtre (23) selon l'axe (40) de la première partie du premier tube étant au moins égale à la longueur hors-tout maximale du dit corps (11) et sa largeur étant déterminée pour permettre l'introduction du corps dans le tube (31).

4. Système selon l'une des revendications 1 à 3, **caractérisé par le fait qu'**il comporte des moyens d'accrochage amovible (24) entre l'extrémité pénétrante (44) de ladite première tige poussoir (43) et ledit corps (11).

5. Système selon la revendication 4, **caractérisé par le fait que** les moyens d'accrochage amovible (24) entre l'extrémité pénétrante (44) de ladite première tige poussoir (43) et ledit corps (11) sont constitués par au moins deux éléments complémentaires détachables, respectivement un élément femelle taraudé et un élément mâle fileté complémentaire apte à être vissé dans ledit élément femelle taraudé, l'un de ces éléments étant monté en coopération avec ledit corps (11) et l'autre avec la première tige poussoir (43).

6. Système selon la revendication 5, **caractérisé par le fait que** ledit élément femelle taraudé est constitué d'un maneton (400) disposé dans une cavité ouverte (402) réalisée dans ledit corps (11), de moyens (401) pour monter ledit maneton en rotation autour d'un axe (403) sensiblement contenu dans le plan longitudinal (15) du corps (11), et d'un orifice taraudé (404) réalisé dans ledit maneton (400), et que ledit élément mâle complémentaire est constitué d'un embout fileté (410) complémentaire du dit orifice taraudé (404), d'un lien (411) souple en flexion et rigide en torsion dont une extrémité est solidaire du dit embout fileté (410), et de moyens (412) montés en coopération avec l'autre extrémité du lien (411) pour commander ledit lien en au moins l'un des deux mouvement suivants : rotation, translation.

7. Système selon l'une des revendications précédentes, **caractérisé par le fait que** les premiers moyens (45) pour commander le déplacement de ladite première tige poussoir (43) dans ledit premier tube (31) sont constitués par des moyens (46) pour entraîner en rotation ladite première tige poussoir (43) par rapport au dit premier tube (31) et des moyens (47) pour transformer le mouvement de rotation de la première tige poussoir (43) en un mouvement de translation.

8. Système selon l'une des revendications précédentes, **caractérisé par le fait que** ledit premier tube (31) comporte en outre au moins une seconde ouverture distale (33-2) réalisée dans la portion de paroi de sa première partie (34-1) qui est prolongée par la portion de paroi intérieure (49) de sa seconde partie (34-2) apte à coopérer par glissement avec la petite base (17) de la seconde partie (12-2) du dit corps (11), la distance (D1) séparant cette seconde ouverture distale (33-2) et la première ouverture distale (33-1) étant sensiblement égale à la longueur hors-tout maximale du dit corps (11).

9. Système selon la revendication 8, **caractérisé par le fait qu'**il comporte en outre un poussoir intermédiaire (55) apte à être introduit entre deux première et seconde cages (10) introduites successivement dans ledit premier tube (31), la distance (D2) entre les première et seconde ouvertures distales (33-1, 33-2) étant sensiblement égale à la somme des longueurs hors-tout maximales du corps (11) et du poussoir intermédiaire (55) prise selon l'axe (40) de la première partie (34-1) du premier tube (31), la face (56) du poussoir intermédiaire (55) apte à venir en contact avec la seconde cage (10) présentant une forme courbe sensiblement congruente de la surface latérale convexe (20) du corps (11).

10. Système selon l'une des revendications précédentes, **caractérisé par le fait que** la seconde partie (34-2) du tube (31) de forme courbe est inscrite dans un dièdre imaginaire (36) d'angle au sommet (α, β) non nul, les deux plans du dièdre (36) se situant respectivement sensiblement dans le plan de jonction (37) des deux première et seconde parties (34-1, 34-2) du premier tube (31) et dans le plan de la première ouverture distale (33-1), son arête étant sensiblement confondue avec un bord de la première ouverture distale, la valeur de l'angle au sommet (α, β) étant comprise entre sensiblement 20 et 90 degrés.

11. Système selon la revendication 1, **caractérisé par le fait que** le ledit guide d'introduction (30) comporte :
* un second tube (131) ayant une seconde ouverture proximale (132-2) et une seconde ouverture distale (133-2), dont la section intérieure transversale est sensiblement complémentaire de la plus grande section transversale hors-tout du dit corps (11) prise dans un plan (19) sensiblement perpendiculaire au dit plan longitudinal (15) et aux deux faces planes (13, 14), ledit second tube (131) comportant au moins deux première et seconde parties consécutives (134-1, 134-2), la première partie (134-1) étant de forme sensiblement rectiligne et comportant la seconde ouverture proximale (132-2), la seconde partie (134-2) étant de forme courbe et comportant la seconde ouverture distale (133-2), la portion de paroi intérieure concave (138) de la seconde partie (134-2) du second tube (131) ayant une courbure sensiblement congruente de celle de la surface latérale convexe (20) de la première partie (12-1) du dit corps (11), la portion de paroi intérieure (149) opposée à la portion de paroi intérieure concave (138), de cette seconde partie (134-2) du second tube ayant une forme apte à coopérer par glissement avec la petite base (17) de la seconde partie (12-2) du dit corps,
* des seconds moyens d'introduction (121) de ladite cage (10) dans la première partie (134-1) du second tube (131) de façon à amener son plan longitudinal (15) sensiblement confondu avec l'axe (140) de ladite première partie (134-1) du second tube (131),
* une seconde tige poussoir (143) apte à pénétrer dans ledit second tube (131) par sa première ouverture proximale (132-2), et
* des seconds moyens (145) pour commander le déplacement de la seconde tige poussoir (143) dans ledit second tube (131),
* les premier et second tubes (31, 131) ayant une partie (190) de paroi latérale commune et étant agencés entre eux de façon que la seconde ouverture distale (133-2) du second tube (131) et la première ouverture distale (33-1) du premier tube (31) soient décalées l'une de l'autre suivant une direction parallèle aux axes longitudinaux (40, 140) des première parties des premier et second tubes d'une valeur (D3) au moins égale à la section hors-tout transversale la plus grande du dit corps (11) prise dans le plan (19) sensiblement perpendiculaire au dit plan longitudinal (15).

12. Système selon la revendication 11, **caractérisé par le fait que** les premiers moyens (45) pour commander le déplacement de ladite première tige poussoir (43) dans ledit premier tube (31) et les seconds moyens (145) pour commander le déplacement de la seconde tige poussoir (143) dans ledit second tube (131) comportent une embase (160), des moyens pour solidariser l'embase (160) avec les extrémités des deux tiges poussoirs (43, 143) opposées à leur extrémité pénétrante (44, 144), et des moyens (180) pour commander la translation de ladite embase (160).

13. Système selon la revendication 12, **caractérisé par le fait que** les moyens (180) pour commander la translation de ladite embase (160) comportent une came poussoir (181), des moyens (182) pour lier en rotation libre une première extrémité (183) de ladite came poussoir (180) avec ladite embase (160), des moyens (184) pour entraîner en rotation ladite came poussoir (181) par rapport aux premier et second tubes (31, 131), et des moyens (185) pour transformer le mouvement de rotation de la came poussoir (181) en un mouvement de translation.

14. Système selon l'une des revendications 1 à 13, **caractérisé par le fait que** l'extrémité pénétrante (44, 144) d'au moins l'une des deux première et seconde tiges poussoirs (43, 143) est agencée pour subir, sur au moins une longueur déterminée non nulle (170), une déformation en courbure.
